# EUROPÄISCHE PATENTSCHRIFT

(11) **EP 1 900 323 B1**
(45) Veröffentlichungstag und Bekanntmachung des Hinweises auf die Patenterteilung: **06.01.2010**
(21) Anmeldenummer: 07017727.4
(22) Anmeldetag: 11.09.2007
(51) Int. Cl.: A61B 5/0408

(54) **Medizinische Elektrode**
Medical electrode
Electrode médicale

(30) Priorität: 15.09.2006 AT 15422006
(43) Veröffentlichungstag der Anmeldung: 19.03.2008
(73) Patentinhaber: Leonh. Lang, 6020 Innsbruck (AT)
(72) Erfinder: Fürtinger, Christian, 6020 Innsbruck (AT)
(74) Vertreter: Torggler, Paul Norbert

(56) Entgegenhaltungen:
- EP-A- 0 635 239
- WO-A-2006/046160
- US-A- 3 989 035
- US-A- 4 522 211
- US-A- 5 632 274

## Beschreibung

Die Erfindung betrifft eine medizinische Elektrode zum Aufbringen auf die Hautoberfläche eines Probanden, mit einem hautseitig haftenden Träger und einem Haltelement für mindestens ein elektrisch leitendes Anschlußstück, wobei das Halbelement mit dem elektrisch leitenden Anschlussstück verklebt ist, wobei das Anschlussstück hautseitig mit einem elektrisch leitenden Gel - insbesondere in einem Schwamm - abgedeckt ist. Weiters betrifft die Erfindung ein Verfahren zur Herstellung einer medizinischen Elektrode.

Nach dem Stand der Technik bekannte, auf die Hautoberfläche eines Probanden aufklebbare, medizinischen Elektroden (zB EKG-Elektroden) bestehen aus einer hautseitig klebenden Trägerschicht mit einer Ausnehmung, in die in der Regel ein elektrisch leitendes Gel (insbesondere eingebettet in einem Schwamm) eingebracht wird. (Die Bezeichnung "Proband" ist an dieser Stelle selbstverständlich vollkommen geschlechtsneutral zu verstehen.) Das Gel steht mit einem elektrisch leitenden Anschlussstück, das häufig auch als Sensorelement oder Eyelet bezeichnet wird, in elektrisch leitendem Kontakt. Gattungsgemäße Elektroden offenbaren zum Beispiel die US 3,989,035 oder die US 4,522,21.1.

Das Anschlussstück kann z.B. aus einem etwa scheibenförmigen Grundkörper mit einem etwa zylinderförmigen Vorsprung bestehen. Der Vorsprung reicht durch eine Ausnehmung im Halteelement während der Grundkörper mit dem Halteelement verklebt ist. Am zylinderförmigen Vorsprung ist das Anschlussstück mit einem elektrischen Leiter, beispielsweise einem Kabel verbunden, um Signale von oder zur Elektrode über beispielsweise einen Elektrokardiographen zu übermitteln. Das Sensorelement ist auf einer Seite (und zwar mit der der Haut zugewandten Seite des scheibenförmigen Grundkörpers) in direktem Kontakt mit dem Gel und auf der gegenüberliegenden Seite also mit der der Haut abgewandten Seite am Halteelement fixiert. Das Halteelement kann z.B. als Etikett vorliegen und am Träger fixiert sein.

Beim Stand der Technik hat es sich als nachteilhaft erwiesen, dass bei Lagerung der medizinischen Elektrode das elektrisch leitende Gel zwischen Anschlussstück und Halteelement eindiffundiert und dabei die Verbindung zwischen den beiden (z.B. in der Form eines Klebers) auflöst. Dadurch kommt es zu einer Beeinträchtigung der Funktion und der Genauigkeit der Elektrode, unter Umständen wird die Elektrode dadurch vollkommen unbrauchbar.

Aufgabe der vorliegenden Erfindung ist es daher, eine medizinische Elektrode der eingangs genannten Gattung so zu verbessern, dass eine längere Haltbarkeit gegeben ist. Weiters soll die mechanische Festigkeit zur Weiterverarbeitung oder Anwendung verbessert werden.

Diese Aufgabe wird mit den Merkmalen des Anspruchs 1 oder mit einem Verfahren nach Anspruch 4 gelöst. Indem der hautseitige Spalt zwischen Halteelement und elektrisch leitendem Anschlussstück (d.h. der Spalt zwischen der Unterseite des Halteelementes und dem elektrisch leitenden Anschlussstück) durch ein, vorzugsweise ringförmiges, Siegelelement überdeckt verschlossen ist bzw. mit dem Siegelelement versiegelt wird, kann das elektrisch leitende Gel nicht mehr in den Spalt zwischen das Halteelement und das Anschlussstück bzw. das Sensorelement eindiffundieren und so das Sensorelement vom Halteelement ablösen.

Dabei hat es sich als vorteilhaft erwiesen, wenn das Siegelelement ein mit einem thermoaktivierbaren Kleber getränktes Vlies ist. Ein thermoaktivierbarer Kleber ist beispielsweise ein so genannter Hotmeltkleber, der durch Temperaturerhöhung verarbeitbar gemacht wird und bei Abkühlen und Erstarren des Klebstoffs eine feste und funktionsfähige Verbindung herstellt. Speziell für die schrittweise Verarbeitung ist ein solches mit einem thermoaktivierbaren Kleber getränktes Vlies vorteilhaft (wie im weiter unten beschriebenen Verfahren noch ausführlich erläutert werden wird) da ein solcher thermoaktivierbarer Kleber mehrfach erhitzt und dadurch verarbeitbar gemacht werden kann.

Die besten Ergebnisse haben sich erzielen lassen, wenn das Sensorelement vollständig von einer leitfähigen Oberfläche bedeckt ist, vorzugsweise Ag/AgCl, wobei weiters günstig ist, wenn der plastifizierbare Grundkörper des Sensorelements ABS umfasst, sowie wenn das Sensorelement Karbonfasern in einer Menge von unter 30 %, vorzugsweise etwa 20 %, aufweist.

Kostengünstiger könnte vorgesehen sein, dass das Siegelelement eine thermoplastische Folie ist, die durch Erwärmen plastifiziert, sodass eine Versiegelung nach dem Erhärten der Folie durch Abkühlung erfolgt.

Weitere Vorteile und Details der Erfindung werden anhand der vorliegenden Zeichnungen näher erläutert. Dabei zeigen
Weiter mit Seite 3 der Beschreibung der Erfindung
- Fig. 1a, 1b und 1c: einen Querschnitt, eine Sicht von oben und eine Sicht von unten auf ein Ausführungsbeispiel einer erfindungsgemäßen medizinischen Elektrode sowie
- Fig. 2: schematisiert Verfahrensschritte eines erfindungsgemäßen Verfahrens.

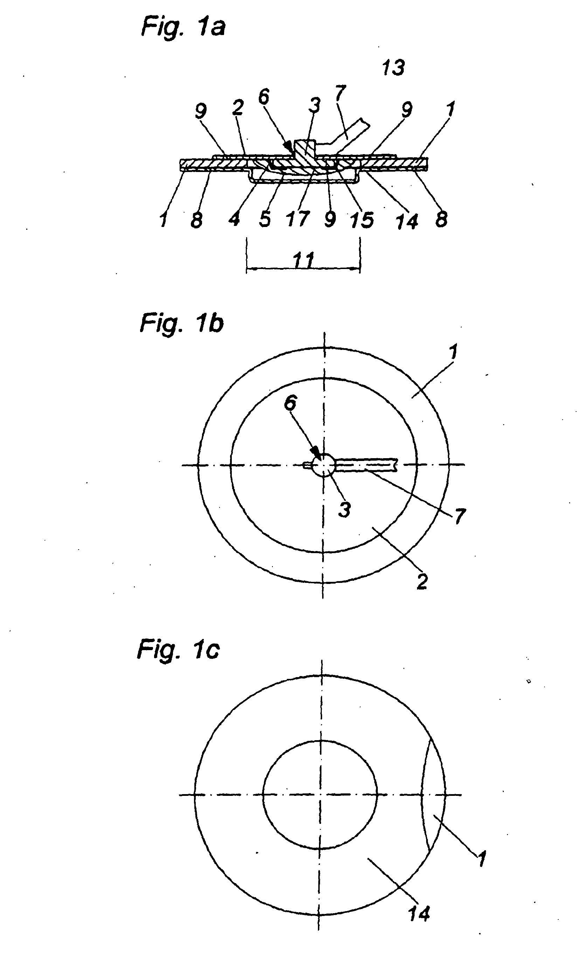

Die Fig. 1a zeigt ein Ausführungsbeispiel einer erfindungsgemäßen medizinischen Elektrode im Querschnitt. Diese Elektrode besteht aus einem Träger 1, der im vorliegenden Fall aus einem Schaumstoff besteht. Dieser Träger 1 weist hautseitig eine Klebeschicht 8 auf, damit die Elektrode auf die (nicht gezeigte) Hautoberfläche aufgeklebt werden kann. Die Kleberschicht 8 sollte hautverträglich ausgebildet sein, damit keine Hautirritationen beim Aufkleben auf die Haut entstehen, ähnlich einem Hautpflaster.

Erkennbar ist außerdem ein Halteelement 2 in der Form eines Etikettes, das mittels eines thermoaktivierbaren Klebers 9 mit dem Träger 1 verbunden ist. Der Träger 1 weist über einen größeren Bereich, wo sich das Haltelement 2 befindet, eine Ausnehmung 11 auf, in die ein elektrisch leitendes Anschlussstück 3 (=Sensorelement, Eyelet) geschoben ist. Erkennbar ist weiters der Schwamm 5, in dem sich ein elektrisch leitendes Gel befindet. Das Gel steht an sich im direkten Kontakt zur Haut und gibt dabei Signale weiter an das Sensorelement 3 bzw. umgekehrt. Schwamm 5, elektrisches Gel und Sensorelement 3 sind alle in der Ausnehmung 11 des Trägers 1 eingebracht und schließen insgesamt etwa bündig mit der Unterseite des Trägers 1 (hautseitig) ab, gegebenenfalls kann, wie gezeigt, der kompressible Schwamm 5 samt elektrischem Gel etwas über den Träger 1 vorstehen, sodass im auf die Haut geklebten Zustand der Schwamm bündig in die Ausnehmung 11 eingepresst wird. Eine Abziehfolie 14 schützt die Elektrode hautseitig vor der Benutzung. Zur Benutzung wird die Abziehfolie entfernt. Der Schwamm 5 ist ebenfalls über den thermoaktivierbaren Kleber 9 mit dem Sensorelement 3 verbunden. Dazu ist ein etwa kreisringförmiger Bereich am Sensorelement 3 mit Kleber 9 bedeckt und der Schwamm 5 wird nach Erwärmen des Klebers 9 aufgesetzt. Nach Erstarren des Klebers ist der Schwamm 5 dauerhaft mit der Elektrode (bzw. dem Sensorelement) verbunden.

Die Außenform des Trägers 1 und die Ausnehmung 11 sind im vorliegenden Fall jeweils etwa kreisförmig ausgebildet, sodass der Träger 1 einen Kreisring bildet. Allerdings ist die Erfindung nicht auf diese Form beschränkt. Beispielsweise sind auch ovale oder eckige Außenformen für den Träger 1 sowie dezentrale, nicht-kreisförmige Ausnehmungen 11 usw. denkbar. Die Form der Ausnehmung 11 hängt im Wesentlichen vom Verwendungszweck und von der Form des Sensorelements 3 bzw. von der Form des Schwammes 5 ab, der in die Ausnehmung 11 eingebracht wird. Träger 1 und Halteelement 2 für das Sensorelement 3 könnten im Rahmen der Erfindung selbstverständlich auch einstückig ausgebildet sein. Das Sensorelement 3 weist dabei einen Grundkörper auf mit dem es mit dem Halteelement 2 ebenfalls über den thermoaktivierbaren Kleber 9 verbunden ist. Das Halteelement 2 weist zusätzlich eine Ausnehmung 6 auf, durch das der etwa zylinderförmige Vorsprung des Sensorelements 3 reicht. An diesem Vorsprung des Anschlussstückes ist der elektrische Leiter 7 (als Kabel mit mehreren Litzen) angebracht der im Betriebszustand die Signale an den nicht gezeigten Elektrokardiographen weiterleitet.

Beim Stand der Technik kann nun im Spaltbereich 15 das elektrisch leitende Gel eindiffundieren und den thermoaktivierbaren Kleber ablösen. Der Spalt 15 befindet sich dabei zwischen der der Hautoberfläche zugewandten Seite des Trägers und der dieser Seite zugewandten, aber der Hautoberfläche abgewandten Seite des Anschlussstückes 3. Zumindest dringt das Gel nach einer gewissen Zeit in den Kleber ein und beeinträchtigt so die Verbindung zwischen Anschlussstück 3 und Halteelement 2. Durch das Einbringen eines im vorliegenden Fall ringförmigen Siegelelementes 4 aus einem mit einem thermoaktivierbaren Kleber getränkten Vlies, lässt sich das Eindiffundieren des elektrisch leitenden Gels vollständig unterbinden. Der Spalt befindet sich dabei zwischen der Unterseite des Halteelementes 3 (d.h. der Seite des Halteelementes 3, die im aufgeklebten oder angebrachten Zustand zur Haut gerichtet ist) und dem elektrisch leitenden Anschlussstück 3. An dieser Stelle sei angemerkt, dass Lageangaben sich auf die in den Figuren dargestellten Normallagen beziehen, bei der die Hautseite unten liegt.

Die Fig. 1b zeigt eine Sicht von oben auf die Elektrode der Fig. 1 a. Erkennbar sind der Träger 1, das Halteelement 2, sowie der zylinderförmige Vorsprung des Anschlussstückes 3, von dem der elektrische Leiter 7 wegführt.

In der Fig. 1c sind in der Sicht von unten auf die Elektrode lediglich der Träger 1 und der Schwamm 5 mit elektrisch leitendem Gel erkennbar. Auf der Elektrode ist die im vorliegenden Fall transparente Abziehfolie 14 angeordnet, die die Elektrode schützt. Eine Ausnehmung erleichtert das Entfernen der Abziehfolie 14.

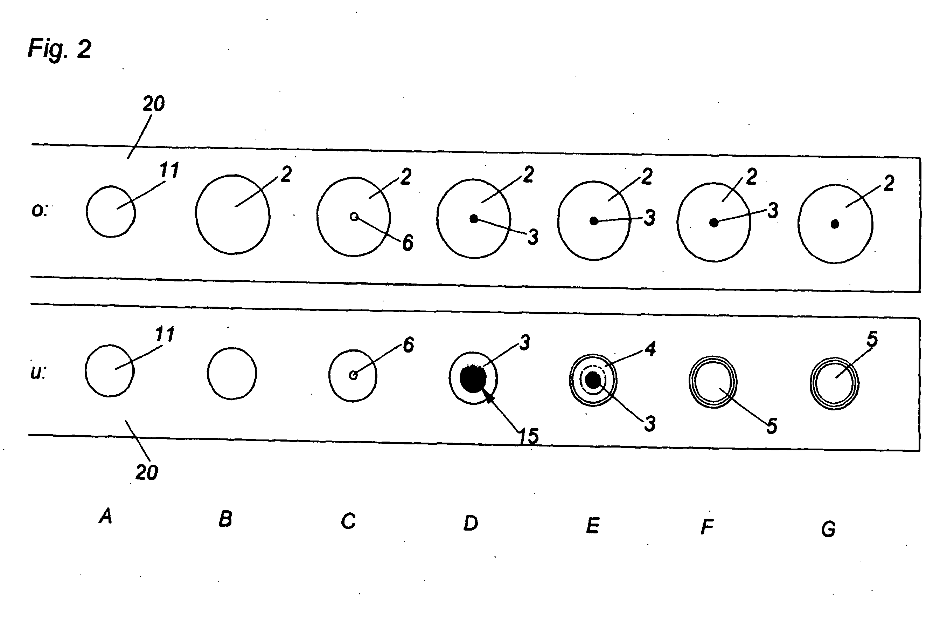

Anhand der Fig. 2 sei an einem Ausführungsbeispiel ein erfindungsgemäßes Verfahren kurz beschrieben. Die Buchstaben A bis H bezeichnen dabei die einzelnen Verfahrensschritte, die Darstellung O beschreibt die Sicht von oben und die Darstellung U die Sicht von unten. A: Im ersten Schritt wird eine Ausnehmung 11 in der Form eines kreisförmiges Loches in ein Bahn 20 die den Träger 1 bildet gestanzt. B: Über die Ausnehmung 11 wird das runde Halteelement 2 in der Form eines Etikettes von oben auf die Bahn 20 geklebt. C: In Schritt 3 wird nun eine kreisförmige Ausnehmung 6, die deutlich kleiner als die Ausnehmung 11 ist, etwa in die Mitte des Halteelementes 2 gestanzt. D: Sodann wird im vierten Schritt ein Sensorelement 3 in die Ausnehmung 6 und mit seinem zylinderförmigen Vorsprung in die Ausnehmung 6 eingesetzt, wobei in der Sicht von oben nur der zylinderförmige Vorsprung erkennbar ist und in der Sicht von unten lediglich der Grundkörper des Anschlussstückes 3 erkennbar ist. Der zylindrische Vorsprung wird durch die Ausnehmung 6 geschoben. E: In Schritt 5 erfolgt nun der sehr wesentliche Schritt, dass nämlich das Siegelelement 4 in der Form eines Fixierringes von unten konzentrisch über das Sensorelement 2 geschoben wird. Durch Erwärmen wird das Siegelement 4 am Halteelement 3 und am Sensorelement 2 fixiert, und zwar versiegelt. F: Anschließend wird der Schwamm 5 über den in die Ausnehmung 11 über das Anschlussstück 3 und das Siegelelement geschoben und ebenfalls mit dem Anschlussstück 3 versiegelt, ebenfalls durch Erwärmen, indem sich auf dem Sensorelement 3 im äußeren Bereich ein thermoaktivierbarer Kleber befindet, der durch Erwärmen verflüssigt und nach Abkühlen eine stabile Verbindung mit dem Schwamm 5 herstellt. An dieser Stelle zeigt sich bereits der Vorteil, wenn als Siegelelement 4 ein Vlies, das in einem thermoaktivierbaren Kleber getränkt wurde, verwendet wird, da durch einfaches Erwärmen der Kleber mehrmals aktiviert werden kann. Im vorliegenden Fall dient das Vlies auf einer Seite als Versiegelung und auf der anderen Seite zur Befestigung des Schwammes 5. G: Im Schritt 7 wird nun der Schwamm 5 mit dem elektrisch leitenden Gel gefüllt. H: Abschließend wird eine Schutz- bzw. Abdeckfolie 14 aufgebracht und danach werden die Elektroden ausgestanzt.

## Patentansprüche

1. Medizinische Elektrode zum Aufkleben auf die Hautoberfläche eines Probanden, mit einem hautseitig haftenden Träger (1) und einem Halteelement (2) für mindestens ein elektrisch leitendes Anschlussstück (3), wobei das Halteelement (2) mit dem elektrisch leitenden Anschlussstück (3) verklebt ist, wobei das Anschlussstück (3) hautseitig mit einem elektrisch leitenden Gel - insbesondere in einem Schwamm - abgedeckt ist, **dadurch gekennzeichnet, dass** der hautseitige Spalt (15) zwischen Halteelement (2) und elektrisch leitendem Anschlussstück (3) durch ein, vorzugsweise ringförmiges, Siegelelement (4) überdeckt und verschlossen ist.

2. Elektrode nach Anspruch 1, **dadurch gekennzeichnet, dass** das Siegelement (4) ein mit einem thermoaktivierbaren Kleber getränktes Vlies ist.

3. Elektrode nach Anspruch 1, **dadurch gekennzeichnet, dass** das Siegelement (4) eine thermoplastische Folie ist.

4. Verfahren zur Herstellung einer medizinischen Elektrode nach einem der Ansprüche 1 bis 3, **dadurch gekennzeichnet, dass** der Spalt (15) zwischen der Unterseite des Halteelementes (2) und dem elektrisch leitenden Anschlussstück (3) mit dem Siegelelement (4) versiegelt wird.

## Claims

1. A medical electrode for placing on the skin of a patient, having a support (1) for attaching to the skin and a holding element (2) for at least one electrically conducting connection piece (3), wherein the holding element (2) is glued to the electrically conducting connection piece (3), wherein the connection piece (3) is covered on the skin side with an electrically conducting gel - in particular in a sponge, **characterized in that** the gap (15) on the skin side between the holding element (2) and the electrically conducting connection piece (3) is covered and closed by a sealing element (4) which is preferably ring-like.

2. An electrode according to claim 1, **characterized in that** the sealing element (4) is a nonwoven impregnated with a heat-activatable adhesive.

3. An electrode according to claim 1, **characterized in that** the sealing element (4) is a thermoplastic film.

4. A process for producing a medical electrode according to one of claims 1 to 3. **characterized in that** the gap (15) between the underside of the holding element (2) and the electrically conducting connection piece (3) is sealed with a sealing element (4).

## Revendications

1. Electrode médicale, à coller sur la surface de la peau d'un patient, avec un support (1), adhérant côté peau, et un élément de maintien (2) pour au moins une pièce de raccordement (3) conductrice de l'électricité, l'élément de maintien (2) étant collé à la pièce de raccordement (3) conductrice de l'électricité, la pièce de raccordement (3) étant couverte, côté peau, par un gel - en particulier dans une éponge - conducteur de l'électricité, **caractérisée en ce que** l'intervalle (15) côté peau, entre élément de maintien (2) et pièce de raccordement (3) conductrice de l'électricité, est couvert et fermé par un élément de scellage (4), de préférence en forme d'anneau.

2. Electrode médicale selon la revendication 1, **caractérisée en ce que** l'élément de scellage (4) est un non tissé imprégné d'un adhésif thermoactivable.

3. Electrode médicale selon la revendication 1, **caractérisée en ce que** l'élément de scellage (4) est une feuille thermoplastique.

4. Procédé de fabrication d'une électrode médicale selon l'une des revendications 1 à 3, **caractérisé en ce que** l'intervalle (15), entre la face inférieure de l'élément de maintien (2) et la pièce de raccordement (3) conductrice de l'électricité, est scellé avec l'élément de scellage (A).
